(19) Europäisches Patentamt
European Patent Office
Office européen des brevets

(11) Veröffentlichungsnummer: **0 160 931**
**A2**

(12) # EUROPÄISCHE PATENTANMELDUNG

(21) Anmeldenummer: 85105317.3

(22) Anmeldetag: 02.05.85

(51) Int. Cl.[4]: C 07 D 249/08
C 07 D 233/62, A 01 N 43/653
A 01 N 43/50

(30) Priorität: 11.05.84 DE 3417468

(43) Veröffentlichungstag der Anmeldung: 13.11.85 Patentblatt 85/46

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Böckmann, Klaus, Dr.
Andreas-Gryphius-Strasse 7
D-5000 Köln 80(DE)

(72) Erfinder: Jäger, Gerhard, Dr.
Gellertstrasse 18
D-5090 Leverkusen 1(DE)

(72) Erfinder: Brandes, Wilhelm, Dr.
Eichendorffstrasse 3
D-5653 Leichlingen 1(DE)

(72) Erfinder: Hänssler, Gerd, Dr.
Heymannstrasse 40
D-5090 Leverkusen 3(DE)

(72) Erfinder: Reinecke, Paul, Dr.
Steinstrasse 8
D-5090 Leverkusen 3(DE)

(72) Erfinder: Stendel, Wilhelm, Dr.
In den Birken 55
D-5600 Wuppertal 1(DE)

(54) **Azolylvinylether.**

(57) Es werden neue Azolylvinylether der allgemeinen Formel

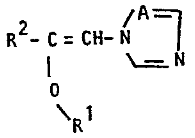

(I)

bereitgestellt,
in welcher
A für ein Stickstoffatom oder die CH-Gruppe steht,
$R^1$ für Alkyl, Alkenyl, Alkinyl, Alkoxyalkyl, gegebenenfalls substituiertes Phenyl, gegebenenfalls substituiertes Phenylalkyl, gegebenenfalls substituiertes Phenoxyalkyl, gegebenenfalls substituiertes Phenoxyalkoxyalkyl, Naphthoxyalkyl, gegebenenfalls substituiertes Cycloalkyl oder gegebenenfalls substituiertes Cycloalkylalkyl steht,
$R^2$ für Alkyl, Halogenalkyl, gegebenenfalls substituiertes Cycloalkyl oder die Gruppierung $R^3-C(CH_3)_2-$ steht, sowie auch für gegebenenfalls substituiertes Phenyl steht, wenn $R^1$ für gegebenenfalls substituiertes Phenoxyalkyl oder für gegebenenfalls substituiertes Phenoxyalkoxyalkyl und A für ein Stickstoffatom stehen und
$R^3$ für jeweils gegebenenfalls substituiertes Phenyl, Phenoxy oder Phenoxyalkyl steht,
und deren Säureadditions-Salze und Metallsalz-Komplexe.
Die Erfindung betrifft weiterhin ein Verfahren zur Herstellung der Verbindungen (I) sowie ihre Verwendung als Fungizide und als Ektoparasitenmittel.

EP 0 160 931 A2

Croydon Printing Company Ltd.

BAYER AKTIENGESELLSCHAFT
Konzernverwaltung RP
Patentabteilung

5090 Leverkusen, Bayerwerk
Slr/AB

## Azolylvinylether

Die vorliegende Erfindung betrifft neue Azolylvinylether, ein Verfahren zu ihrer Herstellung sowie ihre Verwendung als Fungizide und als Ektoparasitenmittel.

Es ist bereits bekannt, daß bestimmte 1-Ethen-azolyl-Derivate, wie beispielsweise 2-(4-Chlorphenoxy)-4,4-dimethyl-1-(imidazol-1-yl)- bzw.-(1,2,4-triazol-1-yl)-1-penten-3-on, gute fungizide Eigenschaften aufweisen (vgl. DE-OS 28 46 980 ).

Außerdem ist bereits bekannt, daß bestimmte Arylvinylazolyl-ether gute fungizide Eigenschaften aufweisen (vgl. DE-OS 27 57 113, DE-OS 28 39 388 und EP-OS 0 079 856).

Weiterhin ist bereits bekannt, daß Disulfide, wie beispielsweise Zinkethylen-1,2-bisdithiocarbamidat, gute Mittel zur Bekämpfung von pilzlichen Pflanzenkrankheiten sind (vgl. R.Wegler, 'Chemie der Pflanzenschutz- und Schädlingsbekämpfungsmittel', Band 2, S. 59 ff, Springer Verlag 1970).

Die Wirkung dieser Verbindungen ist jedoch in bestimmten Indikationsbereichen, insbesondere bei niedrigen Aufwandmengen und -konzentrationen, nicht immer voll befriedigend.

Es wurden neue Azolylvinylether der allgemeinen Formel (I),

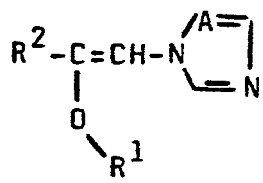

(I)

in welcher

A für ein Stickstoffatom oder die CH-Gruppe steht,

$R^1$ für Alkyl, Alkenyl, Alkinyl, Alkoxyalkyl, gegebenenfalls substituiertes Phenyl, gegebenenfalls substituiertes Phenylalkyl, gegebenenfalls substituiertes Phenoxyalkyl, gegebenenfalls substituiertes Phenoxyalkoxyalkyl, Naphthoxyalkyl, gegebenenfalls substituiertes Cycloalkyl oder gegebenenfalls substituiertes Cycloalkylalkyl steht,

$R^2$ für Alkyl, Halogenalkyl, gegebenenfalls substituiertes Cycloalkyl oder die Gruppierung $R^3$-$C(CH_3)_2$- steht, sowie auch für gegebenenfalls substituiertes Phenyl steht, wenn $R^1$ für gegebenenfalls substituiertes Phenoxyalkyl oder für gegebenenfalls substituiertes Phenoxyalkoxyalkyl und A für ein Stickstoffatom stehen und

$R^3$ für jeweils gegebenenfalls substituiertes Phenyl, Phenoxy oder Phenoxyalkyl steht,

und deren Säureadditions-Salze und Metallsalz-Komplexe gefunden.

Die Verbindungen der Formel (I) können in zwei geometrischen Isomerenformen vorliegen, je nach Anordnung der Gruppen, die an die Doppelbindung gebunden sind; vorzugsweise fallen sie in einem wechselnden Isomerenverhältnis an. Die vorliegende Erfindung betrifft sowohl die einzelnen Isomeren als auch die Isomerengemische.

Weiterhin wurde gefunden, daß man die neuen Azolylvinylether der Formel (I) erhält, wenn man Azolylmethyl-Ketone der Formel (II),

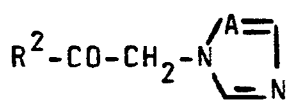

(II)

in welcher

A und $R^2$ die oben angegebene Bedeutung haben,

mit Verbindungen der Formel (III),

$$R^1\text{-}Z \qquad \text{(III)}$$

in welcher

$R^1$ die oben angegebene Bedeutung hat und

Z für eine elektronenanziehende Abgangsgruppierung steht,

in Gegenwart einer starken Base und in Gegenwart eines aprotischen, polaren Verdünnungsmittels, oder in einem wäßrig-organischen Zweiphasensystem in Gegenwart eines Phasentransfer-Katalysators umsetzt.

An die so erhaltenen Verbindungen der Formel (I) kann gegegebenenfalls anschließend eine Säure oder ein Metallsalz addiert werden.

Weiterhin wurde gefunden, daß die neuen Azolylvinylether der Formel (I) starke fungizide Eigenschaften aufweisen und sich außerdem auch zur Bekämpfung von Ektoparasiten auf dem Gebiet der Tierhaltung und Viehzucht eignen.

Überraschenderweise zeigen die erfindungsgemäßen Verbindungen bessere fungizide Wirkungen als die aus dem Stand der Technik bekannten Verbindungen 2-(4-Chlorphenoxy)-4,4-dimethyl-1-(imidazol-1-yl)- bzw.-(1,2,4-triazol-1-yl)-1-penten-3-on und Zinkethylen-1,2-bisdithiocarbamidat. Die erfindungsgemäßen Wirkstoffe stellen somit eine Bereicherung der Technik dar.

Die erfindungsgemäßen Azolylvinylether sind durch die Formel (I) allgemein definiert. In dieser Formel stehen vorzugsweise

A für ein Stickstoffatom oder die CH-Gruppe;

$R^1$ für geradkettiges oder verzweigtes Alkyl mit 1 bis 12 Kohlenstoffatomen, Alkenyl und Alkinyl mit jeweils 2 bis 20 Kohlenstoffatomen, Alkoxyalkyl mit 1 bis 4 Kohlenstoffatomen in jedem Alkylteil, Naphthoxyalkyl mit 1 bis 4 Kohlenstoffatomen im Alkylteil, sowie für jeweils gegebenenfalls einfach oder mehrfach, gleich oder verschieden im Phenylteil substituiertes Phenyl, Phenylalkyl mit 1 bis 4 Kohlenstoffatomen im Alkylteil,

Phenoxyalkyl mit 1 bis 4 Kohlenstoffatomen im Alkylteil und Phenoxyalkoxyalkyl mit 1 bis 4 Kohlenstoffatomen in jedem Alkylteil, wobei jeweils als Phenylsubstituenten genannt seien : Halogen, Alkyl und Alkoxy mit jeweils 1 bis 4 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen, Nitro, Cyano, jeweils gegebenenfalls durch Halogen substituiertes Phenyl, Phenoxy oder Phenylalkyl mit 1 bis 4 Kohlenstoffatomen im Alkylteil, sowie gegebenenfalls durch Alkyl mit 1 bis 4 Kohlenstoffatomen substituiertes Cycloalkyl mit 5 bis 7 Kohlenstoffatomen;

$R^1$ steht ferner vorzugsweise für jeweils gegebenenfalls einfach oder mehrfach, gleich oder verschieden durch Alkyl mit 1 bis 4 Kohlenstoffatomen substituiertes Cycloalkyl oder Cycloalkylalkyl mit jeweils 5 bis 7 Kohlenstoffatomen im Cycloalkylteil und 1 bis 4 Kohlenstoffatomen im Alkylteil;

$R^2$ für geradkettiges oder verzweigtes Alkyl mit 1 bis 8 Kohlenstoffatomen, Halogen-tert.-butyl mit 1oder 2 Halogenatomen, die Gruppierung $R^3$-$C(CH_3)_2$- sowie für gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen; ferner auch für gegebenenfalls einfach oder mehrfach, gleich oder verschieden sustituiertes Phenyl, wobei als Substituenten die bei $R^1$ bereits genannten Phenylsubstituenten infrage kommen, wenn $R^1$ für gegebenenfalls substituiertes Phenoxyalkyl oder gegebenenfalls substituiertes Phenoxyalkoxyalkyl steht und A für ein Stickstoffatom steht;

$R^3$ für jeweils gegebenenfalls einfach oder mehrfach, gleich oder verschieden im Phenylteil substituiertes Phenyl, Phenoxy oder Phenoxyalkyl mit 1 bis 4 Kohlenstoffatomen im Alkylteil steht.

Besondersbevorzugt sind diejenigen Verbindungen der Formel (I), in denen

A für ein Stickstoffatom oder die CH-Gruppe steht;

$R^1$ für geradkettiges oder verzweigtes Alkyl mit 1 bis 8 Kohlenstoffatomen, für Alkenyl mit 2 bis 18 Kohlenstoffatomen und Alkinyl mit 2 bis 6 Kohlenstoffatomen, für Alkoxyalkyl mit 1 bis 4 Kohlenstoffatomen im Alkoxyteil und 1 bis 2 Kohlenstoffatomen im Alkylteil sowie für Naphthoxyalkyl mit 1 bis 2 Kohlenstoffatomen im Alkylteil steht; ferner für jeweils gegebenenfalls einfach bis dreifach, gleich oder verschieden im Phenylteil substituiertes Phenyl, Phenylalkyl mit 1 bis 2 Kohlenstoffatomen im Alkylteil, Phenoxyalkyl mit 1 bis 2 Kohlenstoffatomen im Alkylteil oder Phenoxyalkoxyalkyl mit 1 bis 2 Kohlenstoffatomen in jedem Alkylteil steht, wobei jeweils als Phenylsubstituenten genannt seien :

Fluor, Chlor, Brom, geradkettiges oder verzweigtes Alkyl mit 1 bis 4 Kohlenstoffatomen, Methoxy, Trifluormethyl, Trifluormethoxy, Trifluormethylthio, Nitro, Cyano, jeweils gegebenenfalls durch Chlor oder Fluor substituiertes Phenyl, Phenoxy oder Phenylalkyl mit 1 bis 3 Kohlenstoffatomen im Alkylteil, Cyclopentyl oder Cyclohexyl; sowie für jeweils gegebenenfalls einfach

oder zweifach, gleich oder verschieden durch Methyl, Ethyl und Isopropyl substituiertes Cyclopentyl, Cyclohexyl, Cyclohexylmethyl, Cyclopentylmethyl oder Cyclohexylethyl steht;

$R^2$ für geradkettiges oder verzweigtes Alkyl mit 1 bis 6 Kohlenstoffatomen, für Fluor-tert.-butyl, Chlor-tert.-butyl, 1,1-Bis-(fluormethyl)-ethyl, 1,1-Bis-(chlormethyl)-ethyl, die Gruppierung $R^3-C(CH_3)_2-$ sowie für jeweils gegebenenfalls einfach oder zweifach, gleich oder verschieden durch Methyl, Ethyl und Isopropyl substituiertes Cyclopentyl oder Cyclohexyl steht; ferner auch für gegebenenfalls einfach bis dreifach, gleich oder verschieden substituiertes Phenyl steht, wobei als Substituenten die bei $R^1$ bereits genannten Phenylsubstituenten infrage kommen, wenn $R^1$ für gegebenenfalls substituiertes Phenoxyalkyl oder gegebenenfalls substituiertes Phenoxyalkoxyalkyl steht und A für ein Stickstoffatom steht; und

$R^3$ für jeweils gegebenenfalls einfach bis dreifach, gleich oder verschieden im Phenylteil substituiertes Phenyl, Phenoxy oder Phenoxyalkyl mit 1 bis 2 Kohlenstoffatomen im Alkylteil steht, wobei als Substituenten die bei $R^1$ bereits genannten Phenylsubstituenten infrage kommen.

Bevorzugte erfindungsgemäße Verbindungen sind auch die Additionsprodukte aus Säuren und denjenigen Azolylvinylethern der Formel (I), in denen die Substituenten A, $R^1$ und $R^2$ die Bedeutungen haben, die bereits vorzugsweise für diese Substituenten genannt wurden.

Zu den Säuren, die addiert werden können, gehören vorzugsweise Halogenwasserstoffsäuren, wie z.B. Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Azolylvinylethern der Formel (I), in denen die Substituenten A, $R^1$ und $R^2$ die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Verwendet man beispielsweise 3,3-Dimethyl-1-(1,2,4-triazol-1-yl)-2-butanon und 4-Chlorphenoxyethyl-p-toluolsulfonat in Gegenwart von Natriumhydrid als Ausgangsstoffe, so kann der Reaktionsablauf des erfindungsgemäßen Verfahrens durch das folgende Formelschema wiedergegeben werden :

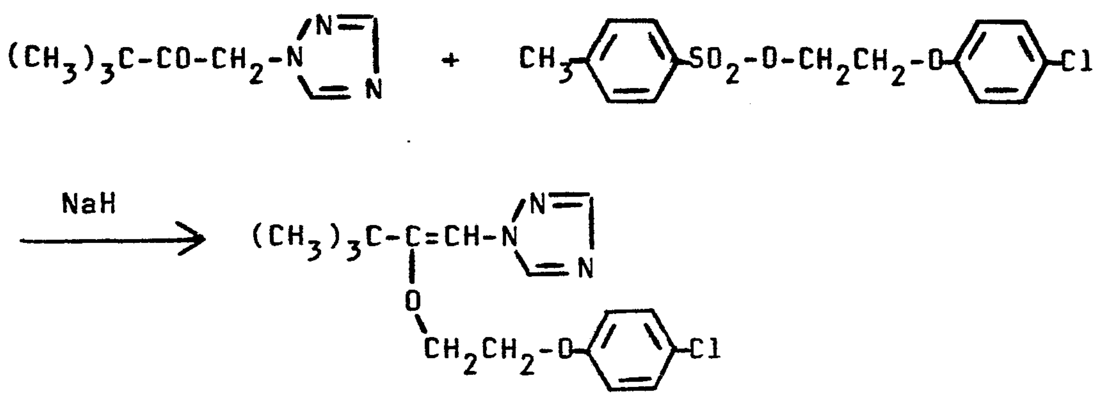


Die bei der Durchführung des erfindungsgemäßen Verfahrens als Ausgangsstoffe zu verwendenden Azolylmethyl-Ketone sind durch die Formel (II) allgemein definiert. In dieser Formel stehen A und $R^2$ vorzugsweise für die Reste, die bereits im Zusammenhang mit der Beschreibung der erfindungsgemäßen Stoffe der Formel (I) vorzugsweise für diese Substituenten genannt wurden.

Die Azolylmethyl-Ketone der Formel (II) sind bekannt (vgl. hierzu z.B. DE-OS 2 431 407, DE-OS 2 610 022, DE-OS 2 638 470, DE-OS 3 145 857 und DE-OS 3 145 858) und werden in allgemein bekannter Art und Weise erhalten, indem man entsprechende Halogenmethyl-Ketone mit Imidazol oder 1,2,4-Triazol in Gegenwart eines Verdünnungsmittels, wie beispielsweise Acetonitril und in Gegenwart eines Säurebinde mittels, wie beispielsweise Kaliumcarbonat,umsetzt.

Die außerdem für das erfindungsgemäße Verfahren als Ausgangsstoffe zu verwendenden Verbindungen sind durch die Formel (III) allgemein definiert. In dieser Formel steht $R^1$ vorzugsweise für diejenigen Reste, die bereits im Zusammenhang mit der Beschreibung der erfindungsgemäßen Stoffe der Formel (I) vorzugsweise für diesen Substituen-

ten genannt wurden. Z steht vorzugsweise für eine elektronenanziehende Abgangsgruppierung, wie beispielsweise Halogen, p-Methylphenylsulfonyloxy, die Gruppierung $-O-SO_2-OR$ oder $-NR^3$ und andere, wobei R hierbei z.B. für Alkyl mit 1 bis 4 Kohlenstoffatomen steht.

Die Verbindungen der Formel (III) sind allgemein bekannte Verbindungen der organischen Chemie.

Für das erfindungsgemäße Verfahren kommen als Verdünnungsmittel aprotische, polare Lösungsmittel infrage. Hierzu gehören vorzugsweise Dimethylformamid, Dimethylacetamid, Dimethylsulfoxid, N-Methylpyrrolidin, 2-Pyrrolidinon und Hexamethylphosphorsäuretriamid. In manchen Fällen kann es vorteilhaft sein, diese Solventien mit anderen üblichen inerten organischen Lösungsmitteln zu mischen, wie beispielsweise aromatische Kohlenwasserstoffe.

Das erfindungsgemäße Verfahren wird in Gegenwart einer starken Base durchgeführt. Hierbei können alle üblichen organischen und insbesondere anorganischen Basen eingesetzt werden, wie vorzugsweise Alkalihydride, -hydroxide oder -carbonate, beispielsweise Natriumhydrid und Natriumhydroxid; tert. Amine, wie Triethylamin, Piperidin und Pyridin.

Die Reaktionstemperaturen können bei der Durchführung des erfindungsgemäßen Verfahrens in einem größeren Bereich variiert werden Im allgemeinen arbeitet man zwischen 0°C und 120°C, vorzugsweise zwischen 20°C und 100°C.

Bei der Durchführung des erfindungsgemäßen Verfahrens setzt man auf 1 Mol Azolylmethyl-Keton der Formel (II) vorzugsweise 1 bis 2 Mol an Verbindung der Formel (III) ein. Die Isolierung der Endprodukte der Formel (I) erfolgt in allgemein üblicher Art und Weise. Dabei werden auch eventuell auftretende Nebenprodukte, die durch Alkylierung an der $CH_2$-Gruppe entstehen, in üblicher Weise, beispielsweise säulenchromatographisch, destillativ oder durch Umkristallisation abgetrennt.

Das erfindungsgemäße Verfahren kann auch in einem Zweiphasensystem, wie beispielsweise wäßrige Natron- oder Kalilauge, Toluol oder Methylenchlorid, gegebenenfalls unter Zusatz von 0,1 bis 1 Mol eines Phasentransferkatalysators, wie beispielsweise Ammonium- oder Phosphoniumverbindungen, beispielsweise seien Benzyldodecyl-dimethylammoniumchlorid und Triethyl-benzyl-ammoniumchlorid genannt, durchgeführt werden.

Die Säureadditionssalze der Verbindungen der Formel (I) können in einfacher Weise nach üblichen Salzbildungsmethoden, z.B. durch Lösen der Verbindung der Formel (I) in einem geeigneten inerten Lösungsmittel und Hinzufügen der Säure, z.B. Chlorwasserstoffsäure, erhalten werden und gegebenenfalls durch Waschen mit einem organischen Lösungsmittel gereinigt werden.

Die Metallsalz-Komplexe von Verbindungen der Formel (I) können in einfacher Weise nach üblichen Verfahren erhalten werden, so z.B. durch Lösen des Metallsalzes in Alkohol,

z.B. Ethanol und Hinzufügen zu Verbindungen der Formel (I). Man kann Metallsalz-Komplexe in bekannter Weise, z.B. durch Abfiltrieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.
Als Pflanzenschutzmittel können die erfindungsgemäßen Wirkstoffe mit besonders gutem Erfolg zur Bekämpfung von Venturia-Arten, wie gegen den Erreger des Apfelschorfes (Venturia inaequalis); von Reiskrankheiten, wie Pyricularia oryzae und Pellicularia sasakii; sowie von Getreidekrankheiten, wie Getreidemehltau, Cochliobolus sativus, Drechslera graminea, Leptosphaeria nodorum und Pyrenophora teres, eingesetzt werden.

Beispielhaft aber nicht begrenzend seien einige Erreger von pilzlichen und bakteriellen Erkrankungen, die unter die oben aufgezählten Oberbegriffe fallen, genannt:

| | |
|---|---|
| Botrytis-Arten, | wie beispielsweise Botrytis cinerea; |
| Plasmopara-Arten, | wie beispielsweise Plasmopara viticola; |
| Uromyces-Arten, | wie beispielsweise Uromyces appendiculatus; |
| Sphaerotheca-Arten, | wie beispielsweise Sphaerotheca fuliginea; |
| Venturia-Arten, | wie beispielsweise Venturia inaequalis; |
| Podosphaera-Arten, | wie beispielsweise Podosphaera leucotrihca; |
| Phytophthora-Arten, | wie beispielsweise Phytophthora infestans; |
| Erysiphe-Arten, | wie beispielsweise Erysiphe graminis; |
| Puccinia-Arten, | wie beispielsweise Puccinia recondita; |
| Fusarium-Arten, | wie beispielsweise Fusarium culmorum |
| Ustilago-Arten, | wie beispielsweise Ustilago nuda oder Ustilago avenae |
| Septoria-Arten, | wie beispielsweise Septoria nodorum; |
| Tilletia-Arten, | wie beispielsweise Tilletia caries; |
| Xanthomonas-Arten, | wie beispielsweise Xanthomonas oryzae; |

| | |
|---|---|
| Pseudomonas-Arten, | wie beispielsweise Pseudomonas lachrymans; |
| Pyricularia-Arten, | wie beispielsweise Pyricularia oryzae, |
| Pellicularia-Arten, | wie beispielsweise Pellicularia sasakii und |
| Pyrenophora-Arten, | wie beispielsweise Pyrenophora teres. (Konidienform Drechslera, Syn. Helminthosporium) |
| Cochliobolus-Arten, | wie beispielsweise Cochliobolus sativus (Konidienform Drechslera, Syn. Helminthosporium) |
| Cercospora-Arten, | wie beispielsweise Cercospora canescens. |

Hervorzuheben ist, daß die erfindungsgemäßen Stoffe nicht nur eine protektive Wirkung entfalten, sondern teilweise auch systemisch sind. So gelingt es, Pflanzen gegen Pilzbefall zu schützen, wenn man die Wirkstoffe über den Boden und die Wurzel oder über das Saatgut den oberirdischen Teilen der Pflanze zuführt.

In entsprechenden Aufwandmengen zeigen die erfindungsgemäßen Stoffe auch wachstumsregulatorische Eigenschaften.

Die erfindungsgemäßen Wirkstoffe eignen sich auch zur Bekämpfung von Ektoparasiten auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesen Gebieten in bekannter Weise, wie durch äußerliche Anwendung in Form beispielsweise des Tauchens

(Dippen), Sprühens (Sprayen), Aufgießens (pour-on and spot-on) und des Einpuderns sowie durch orale Anwendung, beispielsweise über das Futter oder Trinkwasser in Form von beispielsweise Tabletten, Kapseln, Tränken, Granulaten.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfs lösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan

oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden,

wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Oele sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Bei der Verwendung als Ektoparasitenmittel kann der Wirkstoffgehalt der aus den handelsüblichen Formulierungen bereiteten Anwendungsformen in weiten Bereichen variiert werden. Die Wirkstoffkonzentration der Anwendungsformen kann von 0,0000001 bis zu 95 Gew.-% Wirkstoff, vorzugsweise zwischen 0,0001 und 1 Gew.-% liegen.

Herstellungsbeispiele :

Beispiel 1 :

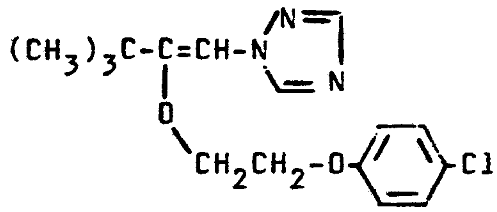

In eine Mischung aus 30 g Natriumhydrid (80 %-ig) und 100 ml trockenem Dimethylformamid tropft man bei Raumtemperatur eine Lösung von 3,3-Dimethyl-1-(1,2,4-triazol-1-yl)-2-butanon in 400 ml trockenem Dimethylformamid zu. Man rührt bei 35°C bis die Lösung klar ist und tropft dann bei Raumtemperatur eine Lösung von 330 g 4-Methylphenylsulfonyl-2-(4-chlorphenoxy)-ethylester in 500 ml trockenem Dimethylformamid zu. Das Reaktionsgemisch wird 12 Stunden bei 40°C gerührt und auf 3 Liter Wasser gegossen. Man extrahiert dreimal mit je 750 ml Essigester, wäscht die vereinigten organischen Phasen dreimal mit je 500 ml Wasser, trocknet über Natriumsulfat und engt im Vakuum ein. Der Rückstand wird destilliert.

Man erhält 253 g (78,8 % der Theorie) 3,3-Dimethyl-2-(4-chlorphenoxyethoxy)-1-(1,2,4-triazol-1-yl)-1-buten vom Siedepunkt 185°C/0,1 mbar.

In entsprechender Weise und gemäß den angegebenen Verfahrensbedingungen können die folgenden Verbindungen der Formel (I) erhalten werden :

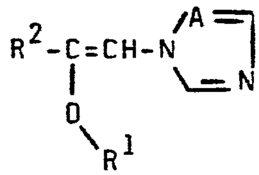

(I)

| Beisp. Nr. | $R^1$ | $R^2$ | A | Fp.(°C) bzw. Kp.(°C)/mbar bzw. $n_D^{20}$ |
|---|---|---|---|---|
| 2 | 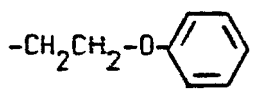 | $(CH_3)_3C-$ | N | 1,5140 |
| 3 | 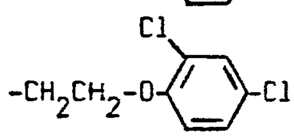 | $(CH_3)_3C-$ | N | 62 - 66 |
| 4 | 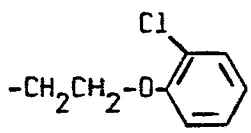 | $(CH_3)_3C-$ | N | 51 |
| 5 | 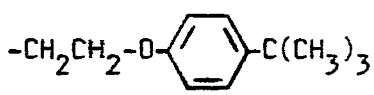 | $(CH_3)_3C-$ | N | 57 - 58 |
| 6 | 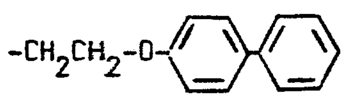 | $(CH_3)_3C-$ | N | 86 - 100 |
| 7 | 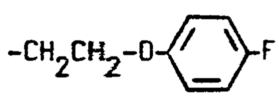 | $(CH_3)_3C-$ | N | 1,5215 |
| 8 | 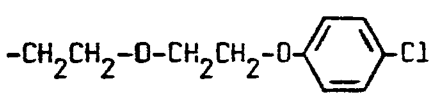 | $(CH_3)_3C-$ | N | 250/0,1 |
| 9 | 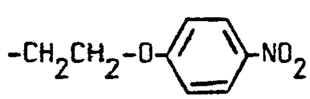 | $(CH_3)_3C-$ | N | 98 |
| 10 | $-C_4H_9-n$ | $(CH_3)_3C-$ | N | 100-105/0,2 |
| 11 | $-CH_2CH_2-O-C_2H_5$ | $(CH_3)_3C-$ | N | 117-121/0,3 |

Fortsetzung

| Beisp. Nr. | $R^1$ | $R^2$ | A | Fp.(°C) bzw. Kp.(°C)/mbar bzw. $n_D^{20}$ |
|---|---|---|---|---|
| 12 | 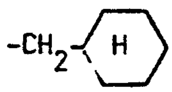 | $(CH_3)_3C-$ | N | 53 |
| 13 | 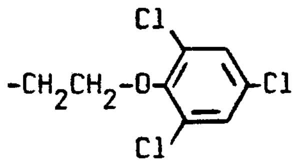 | $(CH_3)_3C-$ | N | 42 |
| 14 | 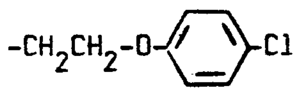 | 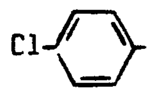 | N | 115 |
| 15 | 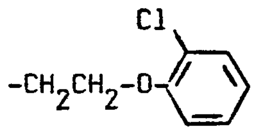 | 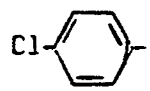 | N | 134 |
| 16 | 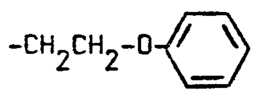 | 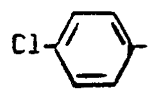 | N | 64 - 66 |
| 17 | 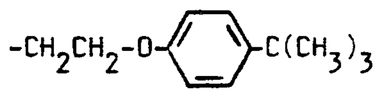 | 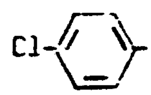 | N | 96 |
| 18 | 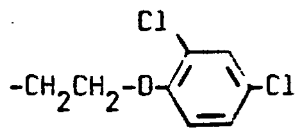 | 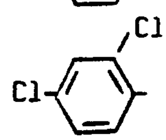 | N | 104 |
| 19 | 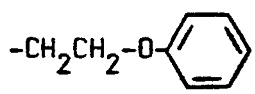 | 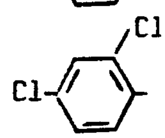 | N | 63 - 64 |
| 20 | 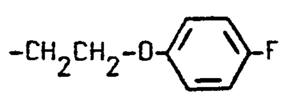 | 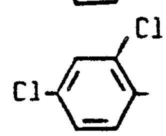 | N | 85 - 89 |
| 21 | 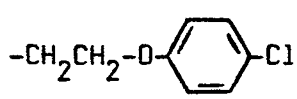 | 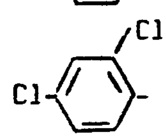 | N | 145 |
| 22 | 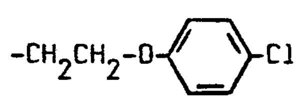 | 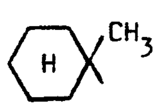 | N | 1,5520 |

Fortsetzung

| Beisp. Nr. | $R^1$ | $R^2$ | A | Fp.(°C) bzw. Kp.(°C)/mbar bzw. $n_D^{20}$ |
|---|---|---|---|---|
| 23 | $-CH_2CH_2-O-$ 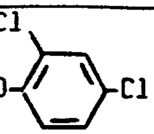 | $(CH_3)_3C-$ | CH | 62 - 66 |
| 24 | $-CH_2CH_2-O-$ 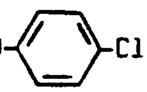 | $(CH_3)_3C-$ | CH | 49 - 52 |
| 25 | $-CH_2CH_2-O-$ 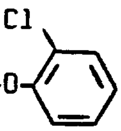 | $(CH_3)_3C-$ | CH | 65 - 70 |
| 26 | $-CH_2CH_2-O-$ 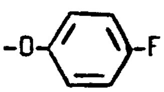 | $(CH_3)_3C-$ | CH | 83 - 85 |
| 27 | $-CH_2CH_2-O-$ 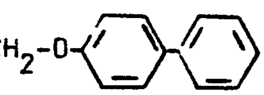 | $(CH_3)_3C-$ | CH | 117-119(Form A)* |
| 28 | $-CH_2CH_2-O-$ 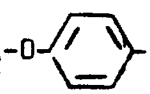 | $(CH_3)_3C-$ | CH | 103 - 105 |
| 29 | $-CH_2CH_2-O-$ 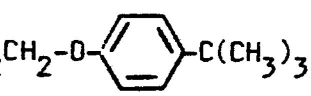 | $(CH_3)_3C-$ | CH | 53 - 60 |
| 30 | $-CH_2CH_2-O-$ 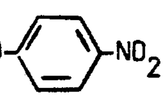 | $(CH_3)_3C-$ | CH | 46 |
| 31 | $-CH_3$ | $(CH_3)_3C-$ | CH | 95/0,2 |
| 32 | $-C_4H_9-n$ | $(CH_3)_3C-$ | CH | 100/0,2 |
| 33 | $-CH_2CH_2-O-C_2H_5$ | $(CH_3)_3C-$ | CH | 112-115/0,3 |
| 34 | $-CH_2-CH(C_2H_5)_2$ | $(CH_3)_3C-$ | CH | 115-118/0,3 |
| 35 | $-CH_2-CH(C_2H_5)-C_3H_7-n$ | $(CH_3)_3C-$ | CH | 124-129/0,3 |
| 36 | $-CH_2(CH_2)_7CH=CH(CH_2)_7CH_3$ | $(CH_3)_3C-$ | CH | 1,4828(Form A)* |
| 37 | $-CH_2(CH_2)_7CH=CH(CH_2)_7CH_3$ | $(CH_3)_3C-$ | CH | 1,4819 |
| 38 | $-CH_2-$ 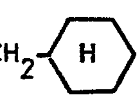 | $(CH_3)_3C-$ | CH | 128-135/0,3 |

Fortsetzung

| Beisp. Nr. | $R^1$ | $R^2$ | A | Fp.(°C) bzw. Kp.(°C)/mbar bzw. $n_D^{20}$ |
|---|---|---|---|---|
| 39 | $-CH_2CH_2-O-$ 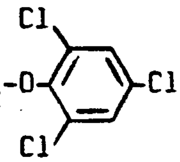 | $(CH_3)_3C-$ | CH | 62 |
| 40 | $-CH_2CH_2-O-$ 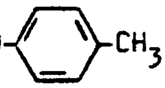 | $(CH_3)_3C-$ | CH | 70 |
| 41 | $-CH_2CH_2-O-$ 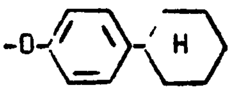 | $(CH_3)_3C-$ | CH | 78 |
| 42 | $-CH_2CH_2-O-$ 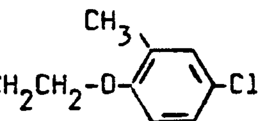 | $(CH_3)_3C-$ | CH | 55 |
| 43 | $-CH_2CH_2-O-$ 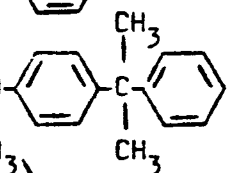 | $(CH_3)_3C-$ | CH | 1,5631 |
| 44 | $-CH_2CH_2-O-$ 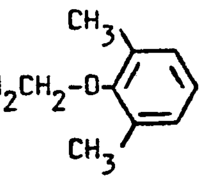 | $(CH_3)_3C-$ | CH | 160/0,2 |
| 45 | $-CH_2CH_2-O-$ 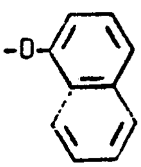 | $(CH_3)_3C-$ | CH | 204/0,1 |
| 46 | $-CH_2CH_2-O-$ 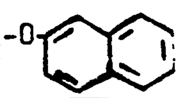 | $(CH_3)_3C-$ | CH | 103 |
| 47 | $-CH_2CH_2-O-$ 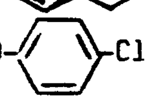 | $(CH_3)_2CH-C(CH_3)_2-$ | CH | 190/0,1 |
| 48 | $-CH_2CH_2-O-$ 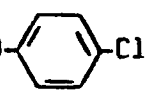 | $FCH_2-C(CH_3)_2-$ | CH | 194/0,1 |

Fortsetzung

| Beip. Nr. | $R^1$ | $R^2$ | A | Fp.(°C) bzw. Kp.(°C)/mbar bzw. $n_D^{20}$ |
|---|---|---|---|---|
| 49 | 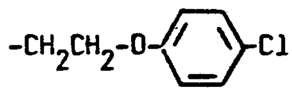 | 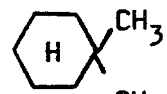 | CH | 1,5310 |
| 50 | 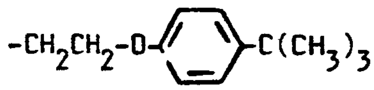 | 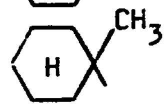 | N | 1,5538 |
| 51 | $-C_4H_9$-n | 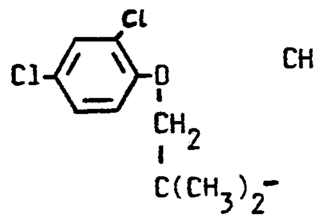 | CH | 1,5392 |
| 52 | 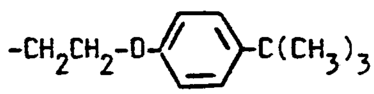 | 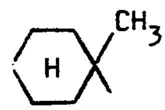 | CH | 1,5395 |

* Form A = eine der beiden möglichen geometrischen Iosmerenformen

Verwendungsbeispiele :

In den nachfolgenden Beispielen werden die nachstehend angegebenen Verbindungen als Vergleichssubstanzen eingesetzt :

(A) 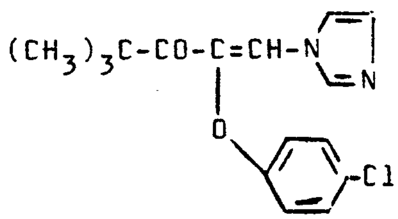

(B) 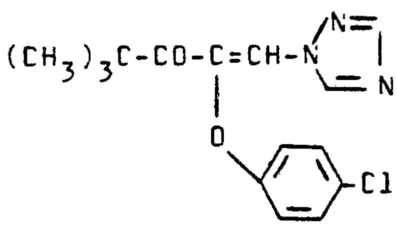

(C) 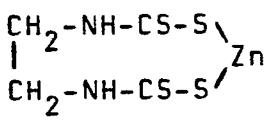

Beispiel A

Venturia-Test (Apfel) / protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 3, 6, 4, 19, 24, 26 und 1.

Beispiel B

Pyricularia-Test (Reis) /protektiv

Lösungsmittel: 12,5 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 23, 24, 25 und 8.

Beispiel C

Pyricularia-Test (Reis) /systemisch

Lösungsmittel: 12,5 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 23, 24, 15, 5 und 10.

Beispiel D

Cochliobolus sativus-Test (Gerste) / protektiv

Lösungsmitel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 1, 16, 5, 18, 7, 23 , 24, 25, 26, 27, 28 und 29.

Beispiel E

Drechslera graminea-Test (Gerste) / Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigen in diesem Test z.B. die Verbindungen gemäß folgender Herstellungsbeispiele: 24 und 26.

Beispiel F

Test an Räudemilben

Screening-Test an Psoroptes ovis/Kontakt-Test in vitro

Testobjekt: Alle Entwicklungsstadien von Psoroptes ovis, die von künstlich infizierten Rindern 1 h vor Testbeginn als Hautgeschabsel entnommen wurden.

Testverfahren: Überführung von 10-25 Milben/Konzentration in 1 ml der zu testenden Wirkstoffkonzentration. Danach Überführung und Aufbewahrung im klimatisierten Testraum (28°C ± 1°C, 80 % rel. Feuchte ± 10 %). Wirkungskontrolle nach 24 Stunden mit dem Stereomikroskop, Vergrößerung 12,5-fach).

Kriterien: Als Wirkungskriterium gilt der Eintritt des Todes bei den behandelten Milben (Todeszeichen = Fehlen willkürlicher Gliedmaßbewegungen nach Reizung mit der Sektionsnadel).

Bewertung: 100 %ige Wirkung = alle Milben sind abgetötet
>50 %ige Wirkung = mehr als 50 % der Milben sind abgetötet

$<$ 50 %ige Wirkung = weniger als 50 % der Milben sind abgetötet

keine Wirkung = alle Milben leben.

In diesem Test zeigen z.B. die Verbindungen gemäß Herstellungsbeispielen 22 und 29 eine sehr gute Wirkung (100 % Abtötung).

Patentansprüche

1. Azolylvinylether der allgemeinen Formel

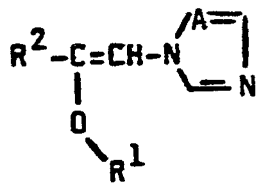

(I)

in welcher

A für ein Stickstoffatom oder die CH-Gruppe steht,

$R^1$ für Alkyl, Alkenyl, Alkinyl, Alkoxyalkyl, gegebenenfalls substituiertes Phenyl, gegebenenfalls substituiertes Phenylalkyl, gegebenenfalls substituiertes Phenoxyalkyl, gegebenenfalls substituiertes Phenoxyalkoxyalkyl, Naphthoxyalkyl, gegebenenfalls substituiertes Cycloalkyl oder gegebenenfalls substituiertes Cycloalkylalkyl steht,

$R^2$ für Alkyl, Halogenalkyl, gegebenenfalls substituiertes Cycloalkyl oder die Gruppierung $R^3-C(CH_3)_2-$ steht, sowie auch für gegebenenfalls substituiertes Phenyl steht, wenn $R^1$ für gegebenenfalls substituiertes Phenoxyalkyl oder für gegebenenfalls substituiertes Phenoxyalkoxyalkyl und A für ein Stickstoffatom stehen und

$R^3$ für jeweils gegebenenfalls substituiertes Phenyl, Phenoxy oder Phenoxyalkyl steht,

und deren Säureadditions-Slaze und Metallsalz-Komplexe.

2. Azolylvinylether der Formel (I) gemäß Anspruch 1), in welcher

A für ein Stickstoffatom oder die CH-Gruppe,

$R^1$ für geradkettiges oder verzweigtes Alkyl mit 1 bis 12 Kohlenstoffatomen, Alkenyl und Alkinyl mit jeweils 2 bis 20 Kohlenstoffatomen, Alkoxyalkyl mit 1 bis 4 Kohlenstoffatomen in jedem Alkylteil, Naphthoxyalkyl mit 1 bis 4 Kohlenstoffatomen im Alkylteil, sowie für jeweils gegebenenfalls einfach bis mehrfach, gleich oder verschieden im Phenylteil substituiertes Phenyl, Phenylalkyl mit 1 bis 4 Kohlenstoffatomen im Alkylteil, Phenoxyalkyl mit 1 bis 4 Kohlenstoffatomen im Alkylteil und Phenoxyalkoxyalkyl mit 1 bis 4 Kohlenstoffatomen in jedem Alkylteil, gegebenenfalls einfach oder mehrfach, gleich oder verschieden durch Alkyl mit 1 bis 4 Kohlenstoffatomen substituiertes Cycloalkyl oder Cycloalkylalkyl mit jeweils 5 bis 7 Kohlenstoffatomen im Cycloalkylteil und 1 bis 4 Kohlenstoffatomen im Alkylteil;

$R^2$ für geradkettiges oder verzweigtes Alkyl mit 1 bis 8 Kohlenstoffatomen, Halogen-tert.-butyl mit 1 oder 2 Halogenatomen, die Gruppierung $R^3-C(CH_3)_2-$ sowie für gegebenenfalls einfach oder mehrfach, gleich oder verschieden durch Alkyl mit 1 bis 4 Kohlenstoffatomen substituiertes Cycloalkyl mit 5 bis 7 Kohlenstoffatomen; ferner auch für gegebenenfalls einfach oder mehrfach, gleich oder verschieden substituiertes Phenyl, wobei als Substituenten die bei $R^1$ bereits genannten Phenylsubstituenten in Frage kommen, wenn $R^1$ für gegebenenfalls substituiertes Phenoxyalkyl oder gegebenenfalls substituiertes Phenoxyalkoxyalkyl steht und A für ein Stickstoffatom steht;

$R^3$ für jeweils gegebenenfalls einfach oder mehrfach, gleich oder verschieden im Phenylteil substituiertes Phenyl, Phenoxy oder Phenoxyalkyl mit 1 bis 4 Kohlenstoffatomen im Alkylteil steht.

3. Azolylvinylether der Formel (I) gemäß Anspruch 1), in welcher

A für ein Stickstoffatom oder die CH-Gruppe steht;

$R^1$ für geradkettiges oder verzweigtes Alkyl mit 1 bis 8 Kohlenstoffatomen, für Alkenyl mit 2

bis 18 Kohlenstoffatomen und Alkinyl mit 2 bis 6 Kohlenstoffatomen, für Alkoxyalkyl mit 1 bis 4 Kohlenstoffatomen im Alkoxyteil und 1 bis 2 Kohlenstoffatomen im Alkylteil sowie für Naphthoxyalkyl mit 1 bis 2 Kohlenstoffatomen im Alkylteil steht; ferner für jeweils gegebenenfalls einfach bis dreifach, gleich oder verschieden im Phenylteil substituiertes Phenyl, Phenylalkyl mit 1 bis 2 Kohlenstoffatomen im Alkylteil, Phenoxyalkyl mit 1 bis 2 Kohlenstoffatomen im Alkylteil oder Phenoxyalkoxyalkyl mit 1 bis 2 Kohlenstoffatomen in jedem Alkylteil steht, wobei jeweils als Phenylsubstituenten genannt seien:

Fluor, Chlor, Brom, geradkettiges oder verzweigtes Alkyl mit 1 bis 4 Kohlenstoffatomen, Methoxy, Trifluormethyl, Trifluormethoxy, Trifluormethylthio, Nitro, Cyano, jeweils gegebenenfalls durch Chlor oder Fluor substituiertes Phenyl, Phenoxy oder Phenylalkyl mit 1 bis 3 Kohlenstoffatomen im Alkylteil, Cyclopentyl oder Cyclohexyl; sowie für jeweils gegebenenfalls einfach oder zweifach, gleich oder verschieden durch Methyl, Ethyl und Isopropyl substituiertes Cyclopentyl, Cyclohexyl, Cylcohexylmethyl, Cyclopentylmethyl oder Cyclohexylethyl steht;

$R^2$ für geradkettiges oder verzweigtes Alkyl mit 1 bis 6 Kohlenstoffatomen, für Fluor-tert.-

butyl, Chlor-tert.-butyl, 1,1-Bis-(fluormethyl)-ethyl, 1,1-Bis-(chlormethyl)-ethyl, die Gruppierung $R^3-C(CH_3)_2-$ sowie für jeweils gegebenenfalls einfach oder zweifach, gleich oder verschieden durch Methyl, Ethyl und Isopropyl substituiertes Cyclopentyl oder Cyclohexyl steht; ferner auch für gegebenenfalls einfach bis dreifach, gleich oder verschieden substituiertes Phenyl steht, wobei als Substituenten die bei $R^1$ bereits genannten Phenylsubstituenten in Frage kommen, wenn $R^1$ für gegebenenfalls substituiertes Phenoxyalkyl oder gegebenenfalls substituiertes Phenoxyalkyl oder gegebenenfalls substituiertes Phenoxyalkoxyalkyl steht und A für ein Stickstoffatom steht; und

$R^3$ für jeweils gegebenenfalls einfach bis dreifach, gleich oder verschieden im Phenylteil substituiertes Phenyl, Phenoxy oder Phenoxyalkyl mit 1 bis 2 Kohlenstoffatomen im Alkylteil steht, wobei als Substituenten die bei $R^1$ bereits genannten Phenylsubstituenten in Frage kommen.

4. Verfahren zur Herstellung von Azolylvinylether der allgemeinen Formel

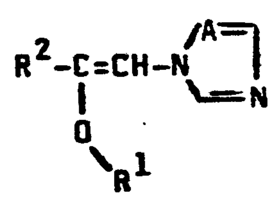

(I)

in welcher

A für ein Stickstoffatom oder die CH-Gruppe steht,

$R^1$ für Alkyl, Alkenyl, Alkinyl, Alkoxyalkyl, gegebenenfalls substituiertes Phenyl, gegebenenfalls substituiertes Phenylalkyl, gegebenenfalls substituiertes Phenoxyalkyl, gegebenenfalls substituiertes Phenoxyalkoxyalkyl, Naphthoxyalkyl, gegebenenfalls substituiertes Cycloalkyl oder gegebenenfalls substituiertes Cycloalkylalkyl steht,

$R^2$ für Alkyl, Halogenalkyl, gegebenenfalls substituiertes Cycloalkyl oder die Gruppierung $R^3-C(CH_3)_2-$ steht, sowie auch für gegebenenfalls substituiertes Phenyl steht, wenn $R^1$ für gegebenenfalls substituiertes Phenoxyalkyl oder für gegebenenfalls substituiertes Phenoxyalkoxyalkyl und A für ein Stickstoffatom stehen und

$R^3$ für jeweils gegebenenfalls substituiertes Phenyl, Phenoxy oder Phenoxyalkyl steht,

und deren Säureadditionssalze und Metallsalzkomplexe, dadurch gekennzeichnet, daß man Azolylmethylketone der Formel

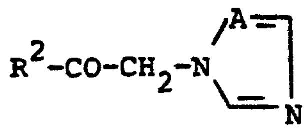

(II)

in welcher

A und $R^2$ die oben angegebene Bedeutung haben,

mit Verbindungen der Formel

$$R^1 - Z \qquad (III)$$

in welcher

$R^1$ die oben angegebene Bedeutung hat und

Z für eine elektronenanziehende Abgangsgruppierung steht,

in Gegenwart einer starken Base und in Gegenwart eines aprotischen, polaren Verdünnungsmittels, oder in einem wäßrigen-organischen Zweiphasensystem in Gegenwart eines Phasentransfer-Katalysators umsetzt, und gegebenenfalls an die so erhaltenen Verbindungen der Formel (I) eine Säure oder ein Metallsalz addiert.

5. Fungizide und ektoparasitizide Mittel, gekennzeichnet durch einen Gehalt an mindestens einem Azolylvinylether der Formel (I).

6. Verfahren zur Bekämpfung von Pflanzenkrankheiten, dadurch gekennzeichnet, daß man Azolylvinylether der Formel I auf Pflanzen oder ihren Lebensraum einwirken läßt.

7. Verfahren zur Bekämpfung von tierischen Ektoparasiten, dadurch gekennzeichnet, daß man Azolylvinylether der Formel (I) anwendet.

8. Verwendung von Azolylvinylethern der Formel (I) zur Bekämpfung von Pflanzenkrankheiten.

9. Verwendung von Azolylvinylethern der Formel (I) zur Bekämpfung von tierischen Ektoparasiten.

10. Verfahren zur Herstellung von fungiziden und ektoparasitiziden Mitteln, dadurch gekennzeichnet, daß man Azolylvinylether der Formel (I) mit Streckmitteln und/oder oberflächenaktiven Mitteln vermischt.